## Europäisches Patentamt

## European Patent Office

## Office européen des brevets

(11) Veröffentlichungsnummer: **0 066 285**
**A1**

(12) **EUROPÄISCHE PATENTANMELDUNG**

(21) Anmeldenummer: **82104741.2**

(51) Int. Cl.³: **G 01 N 33/54**

(22) Anmeldetag: **29.05.82**

(30) Priorität: **03.06.81 DE 3122019**

(43) Veröffentlichungstag der Anmeldung:
**08.12.82 Patentblatt 82/49**

(84) Benannte Vertragsstaaten:
**AT BE CH DE FR GB IT LI LU NL SE**

(71) Anmelder: **Boehringer Mannheim GmbH**
**Sandhoferstrasse 116**
**D-6800 Mannheim 31(DE)**

(72) Erfinder: **Tanswell, Paul, Dr.**
**Eichendorffweg 14**
**D-7959 Obersulmetingen(DE)**

(72) Erfinder: **Reasons, Robert, Dr.**
**24411 Diamante Mission Viejo**
**California, CA 92692(US)**

(54) Verfahren und Vorrichtung zur Durchführung eines automatisierten kontinuierlichen Enzymimmunotestes.

(57) Die Erfindung betrifft ein immunologisches Verfahren zur Bestimmung der Konzentration eines Analyten in einer Probe unter Verwendung eines auf diesen Analyten gerichteten Rezeptors sowie eines Konjugates, das aus dem zu bestimmenden Analyten und einem daran kovalent gekoppelten, geeigneten Enzym besteht. Dabei wird der zu bestimmende Analyt in der Probe mit einer bekannten Menge des Rezeptors und des Konjugats vorinkubiert, das Inkubationsgemisch auf eine Trenneinheit, die ein geeignetes Trennmittel enthält, an das gegen den zu bestimmenden Analyten gerichtete Rezeptoren im Überschuß gebunden sind, aufgebracht, der nicht von dem Trennmittel gebundene Anteil des Inkubationsgemisches eluiert und im Eluat die Enzymaktivität mit Hilfe eines hierfür geeigneten Enzymnachweissystems bestimmt. Ferner wird eine Vorrichtung zur Durchführung des immunologischen Verfahrens beansprucht.

Fig. 1

EP 0 066 285 A1

## Verfahren und Vorrichtung zur Durchführung eines automatisierten kontinuierlichen Enzymimmunotestes

Die Erfindung betrifft ein immunologisches Verfahren zur Bestimmung der Konzentration eines Antigens oder Antikörpers in einer Probe, wobei als Markierungsmittel ein geeignetes Enzym verwendet wird. Insbesondere betrifft die Erfindung einen automatisierten kontinuierlichen Enzymimmunotest (Enzymimmunoassay) zum Messen der Antigen- bzw. Antikörperkonzentration mittels selektiver Antikörper-Antigen-Reaktionen. Die Erfindung betrifft ferner eine Vorrichtung zur Durchführung des enzymimmunologischen Verfahrens.

Auf dem Gebiet der Enzymimmunoassays (EIA) besitzen die sogenannten heterogenen EIAs (beispielsweise ELISA-Test mit antikörperbeschichteten Röhrchen; ELISA = Enzym linked immunosorbent assay) Vorteile gegenüber den sogenannten homogenen Enzymimmunoassays (beispielsweise die EMIT-Technik; EMIT = Enzyme multiplied immunoassay technic). Der heterogene EIA (beispielsweise der ELISA) beinhaltet einen Trennschritt zur Abtrennung des enzymmarkierten Antikörper-Antigen-Komplexes vom freien markierten Antigen bzw. vom freien markierten Antikörper. Die Enzymaktivitätsmessung beim heterogenen EIA erfolgt somit erst nach Entfernung von störenden Serumkomponenten. Es kann daher im allgemeinen wesentlich empfindlicher gemessen werden.

0066285

Bei der manuellen Durchführung eines heterogenen EIA muß die Trennung des gebundenen vom freien Anteil, die sogenannte bound/free-Trennung (B/F-Trennung) in jedem Probengefäß einzeln durchgeführt werden. Sie wird üblicherweise dadurch bewirkt, daß der spezifische Antikörper bzw. das spezifische Antigen an eine Festphase gebunden werden. Die Festphase kann die Probengefäßwand sein, an die der Antikörper bzw. das Antigen direkt oder über ein geeignetes Kupplungsglied gekoppelt sind, oder es können beschichtete Partikel, wie Kugeln, beladene Sepharose- oder Cellulosepartikel sein, die zu jedem Probengefäß einzeln zugegeben werden müssen.

Nach Ablauf der Immunreaktion zwischen immobilisiertem Antikörper bzw. immobilisiertem Antigen und dem freien Antigen bzw. freien Antikörper, die einen bekannten Gehalt an einer in geeigneter Weise enzymmarkierten Form des Antigens bzw. Antikörpers besitzen, wird die Lösung mit dem freien Antigen bzw. Antikörper von dem immobilisierten Antikörper-/Antigenkomplex entfernt. Sowohl in dem in Lösung befindlichen freien Anteil als auch an der Festphase kann die Enzymaktivität bestimmt werden. Anhand der gemessenen Enzymaktivität läßt sich mit Hilfe von Eichkurven, die mit bekannten Konzentrationen des zu bestimmenden Antigens bzw. Antikörpers erhalten worden sind, der Gehalt des zu bestimmenden Antigens bzw. Antikörpers in der unbekannten Probe bestimmen.

Dieser Arbeitsablauf benötigt eine große Anzahl von manuellen Operationen wie Pipettieren, Waschen der Festphase und gegebenenfalls Absaugen oder Zentrifugation im Falle von partikelgebundenen Antikörpern bzw. Antigenen. Werden antigen- bzw. antikörperbeschichtete Formkörper wie z.B. Röhrchen benutzt, so ist darüberhinaus die Herstellung der Formkörper und die Beschichtung mit Antikörpern bzw. Antigenen aufwendig und mit Ungenauigkeiten behaftet. Es ist daher seit geraumer Zeit ein Bestreben, enzymimmunologische Bestimmungen, wie beispielsweise von Hormonen, Pharmaka und ähnlichen Substanzen, zu automatisieren und kontinuierlich durchführbar zu machen. Dadurch soll die Präzision der Bestimmungen erhöht, der Probendurchsatz gesteigert und die Arbeits- und Reagentienkosten gesenkt werden. Die Enzymimmunoassaytechnik bereitet jedoch wegen der zahlreichen im Arbeitsablauf vorhandenen Schritte erhebliche Probleme bei der Automatisierung.

Der Erfindung liegt die Aufgabe zugrunde, ein kontinuierliches, automatisch ablaufendes enzymimmunologisches Bestimmungsverfahren zur schnellen und genauen Bestimmung von Antigenen bzw. Antikörpern durch bestimmte Antikörper-/Antigenwechselwirkungen zu schaffen, wobei Enzyme als Markierungssubstanzen verwendet und geeignete Nachweistechniken für die verwendeten Enzyme eingesetzt werden. Es war ferner Aufgabe der Erfindung, eine geeignete Vorrichtung zur Durchführung des Verfahrens bereitzustellen.

Gelöst wird diese Aufgabe mit dem Verfahren bzw. der Vorrichtung gemäß der vorliegenden Erfindung.

Das erfindungsgemäße Verfahren zeichnet sich durch die folgenden Verfahrensschritte aus:

a) Das zu bestimmende Antigen bzw. der zu bestimmende Antikörper in der Probe - im Folgenden "Analyt" genannt -, wird mit einer bekannten Menge eines spezifischen, gegen das zu bestimmende Antigen bzw. den zu bestimmenden Antikörper gerichteten Antikörpers bzw. Antigens - im Folgenden "Rezeptor" genannt - und einer bekannten Menge eines Konjugats, bestehend aus dem zu bestimmenden Analyten und einem geeigneten, kovalent an den Analyten gekoppelten Enzym vorinkubiert,

b) das Inkubationsgemisch wird auf eine Trenneinheit aufgebracht, die mit einem geeigneten Trennmittel gefüllt ist, an das gegen den zu bestimmenden Analyten gerichtete Rezeptoren im Überschuß gebunden sind;

c) der von dem Trennmittel nicht gebundene Anteil wird mit einem geeigneten Elutionsmittel eluiert und

d) die Enzymaktivität wird im Eluat mit Hilfe eines hierfür geeigneten Enzymnachweissystems bestimmt.

Während der Inkubationszeit konkurrieren der nichtmarkierte, zu bestimmende Analyt und der enzymmarkierte Analyt um die freien Bindungsstellen in den der Probe zugesetzten Rezeptormolekülen, bis eine gewisse Gleichgewichtseinstellung erreicht ist. Das thermische Gleichgewicht wird üblicherweise erst nach längerer Zeit erreicht. Man spricht von dem sogenannten equilibrierten Enzymimmunoassay. Wird jedoch stets eine gleichbleibende Zeitspanne für die Inkubation eingehalten, so ist es nicht erforderlich, das thermische Gleichgewicht abzuwarten, sondern es kann für vergleichbare Tests nach einer bestimmten, experimentell leicht zu ermittelnden Zeit die Inkubation abgebrochen und die weitere Bearbeitung der Probe vorgenommen werden. Die Zeit für die Inkubation bestimmt sich im wesentlichen dadurch, daß einerseits eine hinreichende Menge des Analyten sich mit dem Rezeptor verbunden hat und andererseits die Inkubationszeit nicht zu lange angesetzt wird, um einen raschen Probendurchsatz durch die automatisierte Vorrichtung gewährleisten zu können. Bei dem erfindungsgemäßen Verfahren hat es sich gezeigt, daß im allgemeinen eine Inkubationszeit von 5-20 Minuten, vorzugsweise 10 Minuten ausreichend ist.

Es ist selbstverständlich auch möglich, die Vorinkubation räumlich und zeitlich von der automatisierten Vorrichtung zu trennen. In diesem Falle ist die Vorinkubation zeitlich nicht durch den Trenn- und Meßzyklus beschränkt. Insbesondere bei niedrigen Analytkonzentrationen empfiehlt es sich, über einen längeren Zeitraum, beispielsweise über Nacht, zu inkubieren. Das Inkubationsgemisch wird erst nach abgeschlossener Vorinkubation in die automatisierte Vorrichtung eingebracht.

Das Inkubationsgemisch wird nach der Inkubation der Trenneinheit zugeführt, in welcher der gebundene von dem freien Anteil des Analyten getrennt wird. Die Trenneinheit kann in verschiedener Weise ausgestaltet sein. Es eignet sich hierfür in besonderer Weise eine Chromatographiesäule, die mit einem geeigneten Trennmaterial gefüllt ist. Im Sinne der vorliegenden Erfindung ist als Trennmaterial ganz besonders geeignet ein Immunadsorbens, das aus porösen Glasperlen (CPG = controlled pore glass) besteht, an die derselbe Rezeptor, der bereits in der Vorinkubation benutzt worden ist, in großem Überschuß kovalent gekoppelt ist. Als Trennmittel

0066285

eignen sich ferner mit überschüssigem Rezeptor beladene Membranschichten, beispielsweise aus Polypropylen, Polystyrol, Polymethylmethacrylat oder Papier, sowie Mikropartikel, beispielsweise hydrophile Latexpartikel gemäß der deutschen Patentanmeldung P 30 48 883.6.

Die auf dem Trennmaterial befindlichen überschüssigen Rezeptoren halten beim Durchlauf des Inkubationsgemisches den nicht-gebundenen Analyten zurück, während der während der Inkubation an den Rezeptor gebundene Anteil des Analyten als Antigen-Antikörper-Komplex durch die Säule hindurchläuft. Es war überraschend, daß der vom in Lösung befindlichen Rezeptor gebundene Anteil des enzymmarkierten Analyten nicht von der Immunadsorbenssäule gebunden wird, sondern ungehindert die Rezeptorstellen der Säule zu passieren vermag. Hierin liegt das Vorteilhafte des beanspruchten Verfahrens, d.h. die Immunadsorptionssäule bindet lediglich den freien Anteil des enzymmarkierten Analyten, das ist der Anteil, der bei der Inkubation nicht vom löslichen Rezeptor gebunden worden ist.

Wird nun ein Immunadsorbens mit entsprechend vielen Rezeptorstellen benutzt, so ist auf diese Weise eine mehrmalige Verwendung des Immunadsorbens für eine große Anzahl von Bestimmungen ohne zwischenzeitliche Regenerierung des Immunadsorbensmaterials möglich. Die Rezeptorstellen am Immunadsorbens werden nur allmählich von dem freien Analyten bzw. enzymmarkierten Analyten abgesättigt. Es ist lediglich notwendig, zwischen den einzelnen Proben die Chromatographiesäule mit Puffer zu waschen, um ein Verschleppen von Restmengen einer Probe in die nachfolgende Probe zu vermeiden. Auf diese Weise ist es möglich, mehr als 1000 Bestim-mungen mit derselben Immunadsorbens-Chromatographie-säule durchzuführen.

Das Eluat, das den nicht-adsorbierten Antigen-Antikörper-Komplex in einem bestimmten Verhältnis zwischen markierter und unmarkierter Form enthält, wird in geeigneter Weise gesammelt und in ein Gefäß überführt, das ein für das verwendete Markierungsenzym geeignetes Nachweissystem enthält. In üblicher Weise wird hierzu ein Substrat benutzt, das mit dem verwendeten Markierungsenzym zu reagieren vermag und dabei in eine leicht nachweisbare Form übergeführt wird. Vorzugsweise wird ein Substrat benutzt, das bei der Reaktion mit dem Markierungsenzym in eine Verbindung übergeführt wird, das ein Absorptionsmaximum im sichtbaren Bereich aufweist und somit leicht mit Hilfe eines üblichen Photometers bestimmt werden kann.

Die Enzymaktivität im Säuleneluat wird daher vorzugsweise mit Hilfe eines photometrischen kinetischen Meßverfahrens bestimmt. Die Enzymaktivität steht im umgekehrten Verhältnis zu der Konzentration des zu bestimmenden Analyten in der Probe. Die Messung wird durchgeführt, indem nach dem Vermischen des Säuleneluates mit der Substratlösung zunächst 5-60 Sekunden, vorzugsweise 20-40 Sekunden abgewartet wird und danach die Extinktionsänderung des Substrates infolge der enzymatischen Reaktion kontinuierlich über weitere 10-60 Sekunden gemessen wird.

Der Analyt stellt üblicherweise eine biologisch aktive Substanz, beispielsweise ein Antigen, ein Hapten oder auch einen Antikörper dar. Diese Substanzen befinden sich normalerweise in einer biologischen Flüssigkeit, beispielsweise im Serum. Stellvertretend für die zahlreichen biologisch aktiven Substanzen, die nach dem erfindungsgemäßen Verfahren bestimmt werden können, seien genannt:

Thyroxin, Triiodothyronin, Testosteron, Digoxin,
Digitoxin, Vitamine und Hormone sowie α-Fötoprotein,
carcinoembrionales Antigen oder verschiedene Pharmaka,
wie beispielsweise Phenytoin, Phenobarbital und
Theophyllin.

Als geeigneter Rezeptor für einen bestimmten Analyten
wird vorzugsweise der auf das verwendete Antigen
oder Hapten gerichtete Antikörper eingesetzt. Wird
als Analyt ein Antikörper verwendet, so wird als
Rezeptor das entsprechende Antigen eingesetzt. Bei
dem erfindungsgemäßen Verfahren findet der betreffende Rezeptor in zweifacher Weise Verwendung:
einmal als Rezeptor in der Inkubationslösung und
zum andern als Rezeptor an den festen Träger in
der Chromatographiesäule gebunden.

Als Markierungsenzyme können alle möglichen Enzyme
verwendet werden, die in einfacher Weise an den
zu bestimmenden Analyten gekoppelt werden können
und die nach einer leicht durchführbaren Methode
nachgewiesen werden können. Derartige Enzyme sind
von den manuell durchgeführten Methoden hinreichend
bekannt, vorzugsweise werden bei dem erfindungsgemäßen Verfahren Glucoseoxidase, Peroxidase,
ß-Galactosidase, alkalische Phosphatase und ähnliche eingesetzt. Die Bestimmung dieser Enzyme
in dem Eluat erfolgt in bekannter Weise. Wird
beispielsweise Glucoseoxidase als Markierungsenzym
verwendet, so läßt sich dieses Enzym in üblicher
Weise mit einem Substratgemisch, das Glucose,
Peroxidase und 2,2'-Azino-di(3-äthyl-benzthiazolin-
sulfonsäure) (ABTS) enthält, nachweisen. Die
Extinktionszunahme bei 405 nm wird gemessen.

Das erfindungsgemäße Verfahren kann in vorteilhafter
Weise mit Hilfe der ebenfalls beanspruchten Vorrichtung durchgeführt werden. Diese ist dadurch gekennzeichnet, daß sie

a) mehrere Vorratsgefäße zur Aufnahme der Probelösung mit dem zu bestimmenden Analyten, der Rezeptorlösung, der Konjugatlösung sowie geeigneter Puffer- und Waschflüssigkeiten,

b) eine Inkubationsvorrichtung,

c) eine Trenneinheit zur Trennung des bound/free-Gemisches,

d) ein Gefäß zur Aufnahme des Eluates mit einem für das verwendete Markierungsenzym geeigneten Nachweissystem,

e) Vorrichtung zur Bestimmung der Enzymkonzentration,

f) Verbindungen der einzelnen Einheiten untereinander, Ventile sowie ein geeignetes Steuersystem zur zeitlichen Steuerung der einzelnen Vorgänge enthält.

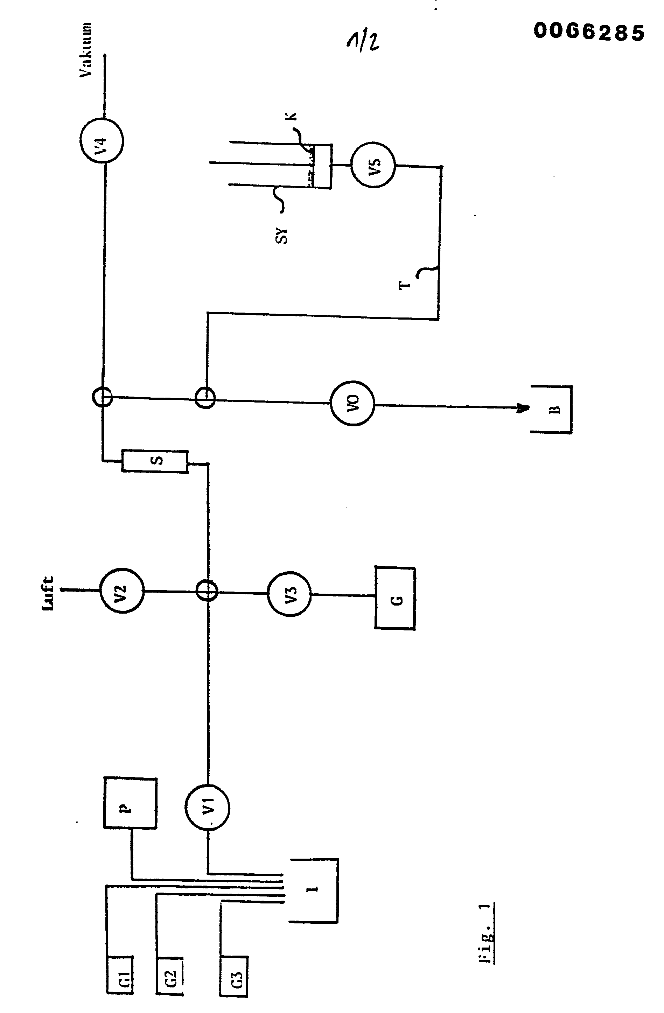

Eine mögliche Ausführungsform der erfindungsgemäßen Vorrichtung ist in Fig. 1 schematisch dargestellt. Aus dem Probegefäß (P), den verschiedenen Vorratsbehältern für Rezeptorlösung (G1), enzymmarkierter Analytlösung (G2) und Pufferlösung (G3) werden entsprechende Proben entnommen und in die Inkubationskammer (I) über die entsprechenden Leitungen transportiert. Das Gemisch wird in dieser Kammer 10 Minuten vorinkubiert. Danach wird durch Öffnen der Ventile V1 und V4 das Inkubationsgemisch auf die Immunadsorbenssäule (S) mittels Vakuum angesaugt. Die Ventile V1 und V4 werden geschlossen. Durch Öffnen der Ventile V3 und V5 und durch Zurückziehen des Kolbens K in einer festmontierten Spritze SY wird die Probe und im Anschluß daran das Verdünnungsmittel (Eluitonspuffer) vom Vorratsgefäß (G) durch
die Säule gezogen. Infolge des dadurch
entstehenden Druckunterschiedes zwischen der
Spritze und dem Säulenauslaß fließt das Eluat
in den Verbindungsschlauch (T) zwischen der
Säule (S) und der Spritze (SY). Sobald das gesamte
Säuleneluat in den Schlauch (T) aufgenommen ist,
wird das Ventil V3 geschlossen, das Ventil VO wird
geöffnet und der motorgetriebene Spritzenkolben
in die entgegengesetzte Richtung bewegt. Dadurch
wird das Säuleneluat aus dem Schlauch (T) in
den Sammelbecher (B) abgestoßen, in dem sich das
zum Nachweis des verwendeten Enzyms erforderliche
Substratgemisch befindet. Das Probensubstratgemisch
wird in die Küvette eines Photometers transportiert,
in dem die enzymatische Nachweisreaktion kinetisch
verfolgt wird. Während der photometrischen Messung
wird in der oben beschriebenen Weise die nächste
Probe behandelt, so daß sich bis zur Beendigung
der photometrischen Messung das Säuleneluat von
der darauffolgenden Probe bereits im Sammelbecher
befindet und der photometrischen Messung seinerseits zugänglich gemacht werden kann. Zweckmäßigerweise findet zwischen den Probenelutionszyklen
jeweils ein kurzer Waschzyklus statt, um sämtliche Leitungen und die Säule von Inkubationsgemischresten der vorangegangenen Probe zu reinigen.
Hierzu wird durch Öffnen der Ventile V3 und V4,
während die übrigen Ventile geschlossen sind, als
Waschflüssigkeit Elutionspuffer aus dem Vorratsgefäß(G)durch die Säule(S)gesaugt. Danach wird durch
Schließen des Ventils V3 und Öffnen des Ventils V2
kurz mit Luft getrocknet.

0066285

Dieser kann so kurz gehalten werden, daß die Gesamtdauer Waschzyklus und Probenelutionszyklus nicht länger ist als die Dauer der photometrischen Messung.

In der beschriebenen Art und Weise ist es möglich, bis zu 45 Proben, vorzugsweise bis zu 35 Proben pro Stunde zu untersuchen. Die erfindungsgemäße Vorrichtung ist nicht auf die hier beschriebene spezielle Ausführungsform beschränkt. Die Erfindung soll sich vielmehr auf alle möglichen Vorrichtungen erstrecken, bei dem das erfindungsgemäße Analysenprinzip in äquivalenter Weise verwirklicht wird. Die erfindungsgemäße Vorrichtung läßt sich nicht nur für solche Verfahren einsetzen, in denen nach der B/F-Trennung eine enzymatische Nachweisreaktion durchzuführen ist, sondern prinzipiell für alle Verfahren, bei denen im Anschluß an eine B/F-Trennung für eine sich darin anschließende Nachweisreaktion ein zusätzliches Reagens dem Säuleneluat beigemischt werden muß.

### Beispiel

### Enzymimmunoassay für Thyroxin ($T_4$)

A **Verwendete Reagentien**

**Thyroxinglucoseoxidase-Konjugat**: Das Thyroxinglucoseoxidase-Konjugat wurde in Analogie zu der deutschen Patentanmeldung P 29 23 139.8 hergestellt. Die Immunreaktivität des eingesetzten Materials betrug 90 % unter den Testbedingungen.

B **Herstellung der Antikörpersäule**

1 ml 3-Aminopropyltriethoxysilan-Glas in Form von Partikeln mit kontrolliertem Durchmesser (80-120 mesh, 2000 A Porengröße), wurde mit 5 ml einer 10-%igen Lösung von Glutaraldehyd 45 Minuten bei 56°C inkubiert. Das aktivierte Glas wurde mehrmals mit 0,15 M Natriumchlorid-Lösung gewaschen und dann mit 2 ml der Immunglobulin-Fraktion eines Schaf-anti-$T_4$-Antiserums 24 Stunden bei Raumtemperatur in einem Rotationsmischer reagieren gelassen. Danach wurden die mit $T_4$-Antikörpern beschichteten Glaspartikel sukzessiv mit 2 M Äthanolamin, pH 8, 3 Stunden lang, mehrmals mit 0,15 M Natriumchlorid-Lösung und danach abwechselnd je 3 mal mit 0,1 M Acetatpuffer, pH 4,0 und 0,1 M Boratpuffer, pH 8,5 gewaschen. Das so gewonnene Immunadsorbensmaterial wurde auf eine Chromatographiesäule $0,7 \times 8 \text{ cm}^2$ (Panchem K12-S) aus Polypropylen mit einer Fritte von 40 µm Porendurchmesser gegeben.

C Durchführung des Enzymimmunoassays

30 μl Humanserum, das entweder als Standard eine genau definierte Menge Thyroxin enthielt oder als unbekannte Probe einem Patienten entstammte, wurden mit 30 μl einer 0,5 M Natriumhydroxid-Lösung 5 Minuten inkubiert. Danach wurde sofort mit 30 μl 0,5 M Salzsäure neutralisiert. Es wurden anschließend 340 μl einer Lösung von $T_4$-Glucoseoxidase-Enzymkonjugat in 0,05 M Phosphatpuffer, pH 7,2, die 0,1 % Rinderserumalbumin enthielt, zugegeben. Die Lösung entsprach einer Glucoseoxidase-Aktivität von 0,025 U. Sofort anschließend wurden 100 μl einer anti-$T_4$-Antikörper-lösung in demselben Puffer zugesetzt. Die Antikörperlösung wurde so verdünnt, daß sie in Abwesenheit von Probe oder Standard ca. 50 % der eingesetzten Glucoseoxidase-Aktivität zu binden vermag.

Dieses Gemisch wurde bei Raumtemperatur 10 Minuten inkubiert und danach in das Trennsystem aufgezogen. Im Anschluß an die Inkubationslösung wurde der Säulenelutionspuffer zugeführt. Dieser war identisch mit dem im Inkubationsgemisch verwendeten Verdünnungspuffer. Das Säuleneluat (1 ml) wurde von der motorgetriebenen Spritze in den Schlauch (T) gezogen und dann in den Sammelbecher ausgestoßen. Der Sammelbecher enthielt 0,5 ml des Substratgemisches, das aus 150 g D-Glucose, 3 g ABTS (2,2'-Azino-di(3-äthyl-benzthiazolinsulfonsäure), Diammoniumsalz), 180 mg Meerretich-Peroxidase in 1 l Phosphatpuffer, pH 6,5 besteht. Nach 50 Sekunden Wartezeit wurde die Extinktionszunahme bei 405 nm über einen Zeitraum von 40 Sekunden gemessen und auf Extinktion/min. umgerechnet. Während dieser 90 Sekunden wurde das Säulen-Trennsystem durch das Einziehen von Puffer gereinigt und auf das nächste Probeninkubationsgemisch vorbereitet.

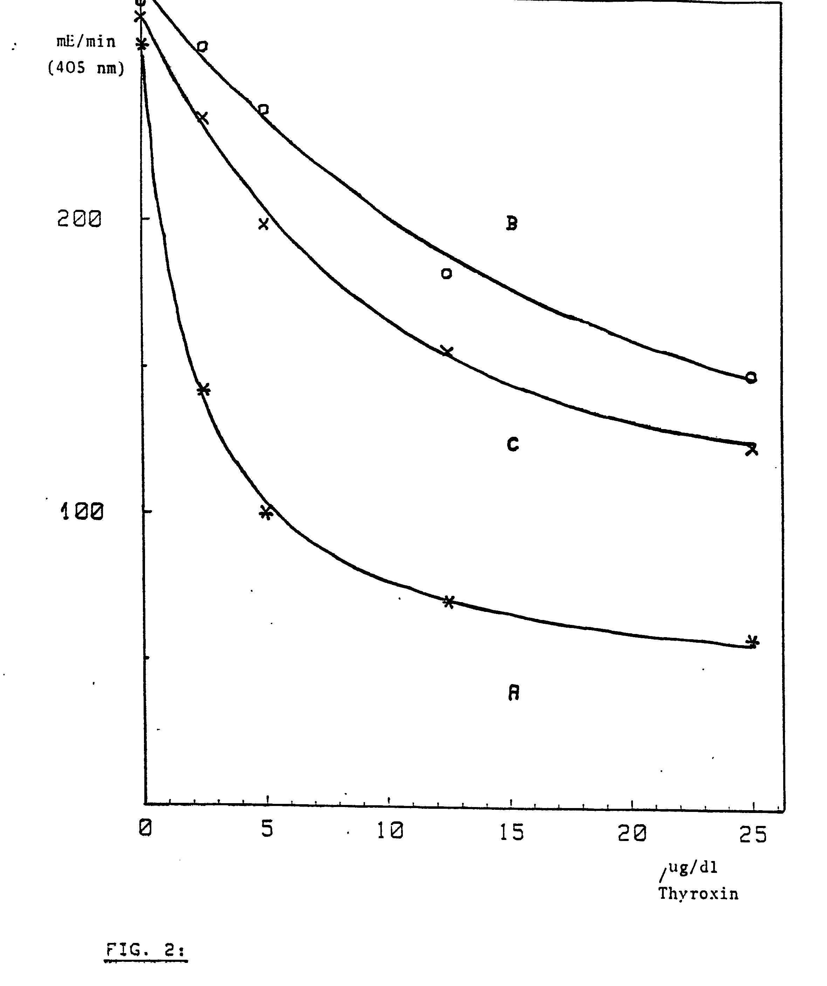

In Fig. 2 ist die aus Messungen mit bekannten Proben resultierende Standardkurve wiedergegeben. Es wird die Extinktion/min. gegen Thyroxinkonzentration in der Probe aufgetragen. Es sind Standardkurven aufgestellt für Serumproben von 20 /ul (Kurve B), 30 /ul (Kurve C) und 50 /ul (Kurve A). Durch Vergleich der für eine unbekannte Probe gefundenen Extinktion mit der Standardkurve kann der unbekannte Thyroxingehalt einer Probe ermittelt werden.

Die beigefügten Figuren zeigen im Einzelnen:

Fig. 1 Schematische Darstellung einer erfindungsgemäß verwendbaren Vorrichtung;

Fig. 2 Standardkurven zur Bestimmung von Thyroxin nach dem erfindungsgemäßen Verfahren. A = 50 /ul Serumprobe; B = 20 /ul Serumprobe; C = 30 /ul Serumprobe.

Patentansprüche

1. Immunologisches Verfahren zur Bestimmung der Konzentration eines Analyten in einer Probe unter Verwendung eines auf diesen Analyten gerichteten Rezeptors sowie eines Konjugates, das aus dem zu bestimmenden Analyten und einem daran kovalent gekoppelten, geeigneten Enzym besteht, dadurch gekennzeichnet, daß

a) der zu bestimmende Analyt in der Probe mit einer bekannten Menge des Rezeptors und des Konjugats vorinkubiert,

b) das Inkubationsgemisch auf eine Trenneinheit, die ein geeignetes Trennmittel enthält, an das gegen den zu bestimmenden Analyten gerichtete Rezeptoren im Überschuß gebunden sind, aufgebracht,

c) der nicht von dem Trennmittel gebundene Anteil des Inkubationsgemisches eluiert und

d) im Eluat die Enzymaktivität mit Hilfe eines hierfür geeigneten Enzymnachweissystems bestimmt wird.

2. Verfahren gemäß Anspruch 1, dadurch gekennzeichnet, daß als Analyt ein Antigen oder Antikörper, als Rezeptor ein gegen den zu bestimmenden Analyten gerichteter spezifischer Antikörper bzw. spezifisches Antigen und als Konjugat der zu bestimmende, mit einem Enzym markierte Analyt verwendet wird.

3. Verfahren gemäß Ansprüchen 1 und 2, dadurch gekennzeichnet, daß als Markierungsenzym Glucoseoxidase, Peroxidase oder ß-Galactosidase eingesetzt wird.

4. Verfahren gemäß Anspruch 2, dadurch gekennzeichnet, daß als Antigen Thyroxin, als Antikörper Thyroxin-Antikörper und als Konjugat Thyroxin-Glucoseoxidase eingesetzt wird.

5. Verfahren gemäß einem der Ansprüche 1-4, dadurch gekennzeichnet, daß von Probe zu Probe eine gleichbleibende Inkubationszeit eingehalten wird.

6. Verfahren gemäß Anspruch 5, dadurch gekennzeichnet, daß die Inkubationszeit 5-20 Minuten beträgt.

7. Verfahren gemäß einem der Ansprüche 1-6, dadurch gekennzeichnet, daß als Trenneinheit eine Chromatographiesäule eingesetzt wird, die mit einem Immunadsorbens aus porösen Glasperlen, an die der Rezeptor gebunden ist, gefüllt ist.

8. Verfahren gemäß einem der Ansprüche 1-6, dadurch gekennzeichnet, daß als Trenneinheit eine Chromatographiesäule eingesetzt wird, die als Trennmittel mit überschüssigem Rezeptor beladene Membranschichten oder Mikropartikel enthält.

9. Vorrichtung zur Durchführung des immunologischen Verfahrens gemäß einem der Ansprüche 1-8, dadurch gekennzeichnet, daß sie

a) mehrere Vorratsgefäße zur Aufnahme der Probelösung mit dem zu bestimmenden Analyten, der Rezeptorlösung, der Konjugatlösung sowie
geeigneter Puffer- und Waschflüssigkeiten,

b) eine Inkubationsvorrichtung,

c) eine Trenneinheit zur Trennung des bound/free-
Gemisches,

d) ein Gefäß zur Aufnahme des Eluates mit einem
für das verwendete Markierungsenzym geeigneten
Nachweissystem,

e) Vorrichtung zur Bestimmung der Enzymkonzentration,

f) Verbindungen der einzelnen Einheiten untereinander, Ventile sowie ein geeignetes Steuersystem zur zeitlichen Steuerung der einzelnen
Vorgänge enthält.

10. Vorrichtung gemäß Anspruch 9, dadurch gekennzeichnet, daß die Trenneinheit eine Chromatographiesäule darstellt, die als Trennmittel
ein Immunadsorbens aus porösen Glasperlen,
Membranschichten oder Mikropartikeln enthält,
die jeweils mit überschüssigem Rezeptor beladen
sind.

Fig. 1

FIG. 2:

**Europäisches Patentamt**

# EUROPÄISCHER RECHERCHENBERICHT

Nummer der Anmeldung

EP 82 10 4741

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-4 104 026 (BROOKER et al.) * Zusammenfassung; Spalte 9, Zeilen 14-25; Spalte 11, Zeilen 30-43, Spalte 13, Zeile 29 - Spalte 14, Zeile 64; Tabelle 1; Ansprüche 1,2,4,6,9,10,12,13 * | 1-10 | G 01 N 33/54 |
| | --- | | |
| Y | US-A-4 108 976 (M. REESE)  * Zusammenfassung; Ansprüche 1,5 * | 1-3,7-10 | |
| | --- | | |
| Y | EP-A-0 006 998 (BOEHRINGER MANNHEIM GmbH) * Zusammenfassung; Beispiel 1; Ansprüche 1-4,7; Seite 5, Zeilen 5-15 * | 1-4,7,8 | |
| | --- | | RECHERCHIERTE SACHGEBIETE (Int. Cl. ³) |
| Y | DE-A-2 736 527 (BOEHRINGER MANNHEIM GmbH) * Ansprüche 1,8,9; Seite 8 - Seite 10, Zeile 26; Beispiel 2 * | 1-4,7-10 | G 01 N |
| | --- | | |
| Y | DE-A-2 804 657 (THE RECTOR & VISITORS OF THE UNIVERSITY OF VIRGINIA) * Ansprüche 1,8,10; Seite 11, Zeilen 27-33; Seite 12, Zeilen 31-36; Seite 13, Zeile 25; Beispiel 6 * | 1-4,7-10 | |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 10-09-1981 | OSBORNE H.H. |